# EUROPÄISCHE PATENTSCHRIFT

(11) **EP 0 686 026 B1**
(45) Veröffentlichungstag und Bekanntmachung des Hinweises auf die Patenterteilung: **03.09.1997**
(21) Anmeldenummer: 94904978.7
(22) Anmeldetag: 29.01.1994
(51) Int. Cl.: A61K 7/48, A61K 7/06, A61K 7/42, A61K 31/34

(54) **SYNERGISTISCHE LICHTSCHUTZKOMBINATIONEN UND KOSMETISCHE UND DERMATOLOGISCHE FORMULIERUNGEN, SOLCHE KOMBINATIONEN ENTHALTEND**
SYNERGISTIC COMBINATIONS OF SUNSCREENING SUBSTANCES, COSMETIC AND DERMATOLOGICAL COMPOSITIONS CONTAINING SUCH COMBINATIONS
COMBINAISONS PHOTOPROTECTRICES SYNERGIQUES ET FORMULATIONS COSMETIQUES ET DERMATOLOGIQUES LES CONTENANT

(30) Priorität: 25.02.1993 DE 4305788
(43) Veröffentlichungstag der Anmeldung: 13.12.1995
(73) Patentinhaber: Beiersdorf Aktiengesellschaft, D-20245 Hamburg (DE)
(72) Erfinder: GERS-BARLAG, Heinrich, D-25493 Kummerfeld (DE); MÜLLER, Anja, D-20535 Hamburg (DE); SAUERMANN, Gerhard, D-24649 Wiemersdorf (DE); UHLMANN, Beate, D-22453 Hamburg (DE)
(86) Internationale Anmeldenummer: DE9400078
(87) Internationale Veröffentlichungsnummer: WO9418942

(56) Entgegenhaltungen:
- EP-A- 0 345 362
- FR-A- 2 233 998
- INTERNATIONAL JOURNAL OF COSMETIC SCIENCE Bd. 12, Nr. 1 , Februar 1990 , LONDON Seiten 1 - 4 'FURAL GLUCITOL AS FREE RADICAL SCAVENGER IN THE PROCESS OF SKIN AGEING'
- AGEING'

## Beschreibung

Die vorliegende Erfindung betrifft Lichtschutzformulierungen, insbesondere kosmetische und dermatologische Lichtschutzmittel.

Die schädigende Wirkung des ultravioletten Teils der Sonnenstrahlung auf die Haut ist allgemein bekannt. Während Strahlen mit einer Wellenlänge, die kleiner als 290 nm ist (der sogenannte UVC-Bereich), von der Ozonschicht in der Erdatmosphäre absorbiert werden, verursachen Strahlen im Bereich zwischen 290 nm und 320 nm, dem sogenannten UVB-Bereich, ein Erythem, einen einfachen Sonnenbrand oder sogar mehr oder weniger starke Verbrennungen.

Als ein Maximum der Erythemwirksamkeit des Sonnenlichtes wird der engere Bereich um 308 nm angegeben.

Zum Schutz gegen UVB-Strahlung sind zahlreiche Verbindungen bekannt, bei denen es sich um Derivate des 3-Benzylidencamphers, der 4-Aminobenzoesäure, der Zimtsäure, der Salicylsäure, des Benzophenons sowie auch des 2-Phenylbenzimidazols handelt.

Auch für den Bereich zwischen etwa 320 nm und etwa 400 nm, den sogenannten UVA-Bereich, ist es wichtig, Filtersubsubstanzen zur Verfügung zu haben, da auch dessen Strahlen Schäden hervorrufen können. So ist erwiesen, daß UVA-Strahlung zu einer Schädigung der elastischen und kollagenen Fasern des Bindegewebes führt, was die Haut vorzeitig altern läßt, und daß sie als Ursache zahlreicher phototoxischer und photoallergischer Reaktionen zu sehen ist. Der schädigende Einfluß der UVB-Strahlung kann durch UVA-Strahlung verstärkt werden.

Zum Schutz gegen die Strahlen des UVA-Bereichs werden daher gewisse Derivate des Dibenzoylmethans verwendet, deren Photostabilität (Int. J. Cosm. Science 10, 53 (1988)) nicht in ausreichendem Maße gegeben ist.

Die UV-Strahlung kann aber auch zu photochemischen Reaktionen führen, wobei dann die photochemischen Reaktionsprodukte in den Hautmetabolismus eingreifen.

Vorwiegend handelt es sich bei solchen photochemischen Reaktionsprodukten um radikalische Verbindungen, beispielsweise Hydroxyradikale. Auch undefinierte radikalische Photoprodukte, welche in der Haut selbst entstehen, können aufgrund ihrer hohen Reaktivität unkontrollierte Folgereaktionen an den Tag legen. Aber auch Singulettsauerstoff, ein nichtradikalischer angeregter Zustand des Sauerstoffmoleküls kann bei UV-Bestrahlung auftreten, ebenso kurzlebige Epoxide und viele Andere. Singulettsauerstoff beispielsweise zeichnet sich gegenüber dem normalerweise vorliegenden Triplettsauerstoff (radikalischer Grundzustand) durch gesteigerte Reaktivität aus. Allerdings existieren auch angeregte, reaktive (radikalische) Triplettzustände des Sauerstoffmoleküls.

Um diesen Reaktionen vorzubeugen, können den kosmetischen bzw. dermatologischen Formulierungen zusätzlich Antioxidantien und/oder Radikalfänger einverleibt werden.

Die teilweise vorstehend genannten Verbindungen, welche als Lichtschutzmittel für kosmetische und dermatologische Lichtschutzformulierungen eingesetzt werden, zeichnen sich an sich durch gute Lichtschutzwirkung aus. Sie haben jedoch den Nachteil, daß es bisweilen schwierig ist, sie in befriedigender Weise solchen Formulierungen einzuverleiben. Ferner sind diese Verbindungen reine UV-Absorber und als Radikalfänger ungeeignet.

Es ist bereits vorgeschlagen worden, Vitamin E, eine Substanz mit bekannter antioxidativer Wirkung in Lichtschutzformulierungen einzusetzen, dennoch bleibt auch hier die erzielte Wirkung weit hinter der erhofften zurück.

Es war indes überraschend und für den Fachmann nicht vorauszusehen, daß
Wirkstoffkombinationen, bestehend aus einer Kombination aus mindestens einer Substanz, gewählt aus der Gruppe aus 2,4-O-Furfurylidensorbitol und dessen Alkylethern sowie mindestens einer Substanz, gewählt aus der Gruppe der kosmetisch oder Pharmazeutisch akzeptablen Antioxidantien sowie
Kosmetische und dermatologische Lichtschutzformulierungen mit einem wirksamen Gehalt an einer Kombination aus mindestens einer Substanz, gewählt aus der Gruppe 2,4-O-Furfurylidensorbitol und dessen Alkylether sowie mindestens einer Substanz,gewählt aus der Gruppe der kosmetisch oder pharmazeutisch akzeptablen Antioxidantien insbesondere aber
Wirkstoffkombinationen, bestehend aus einer Kombination aus mindestens einer Substanz, gewählt aus der Gruppe aus 2,4-O-Furfurylidensorbitol und dessen Alkylethern sowie mindestens einer Substanz, gewählt aus der Gruppe der Tocopherole und Tocopherylester
   sowie
Kosmetische und dermatologische Lichtschutzformulierungen mit einem wirksamen Gehalt an einer Kombination aus mindestens einer Substanz, gewählt aus der Gruppe 2,4-O-Furfurylidensorbitol und dessen Alkylether sowie mindestens einer Substanz, gewählt aus der Gruppe der Tocopherole und Tocopherylester, den Nachteilen des Standes der Technik abhelfen.

2,4-O-Furfurylidensorbitol ist synonym mit den Bezeichnungen 2,4-O-Furfurylidenglucitol, 2,4-O-(2-Furanylmethylen)-Glucitol, 2,4-Monofurfurylidensorbitol. Es zeichnet sich durch die folgende Struktur aus: 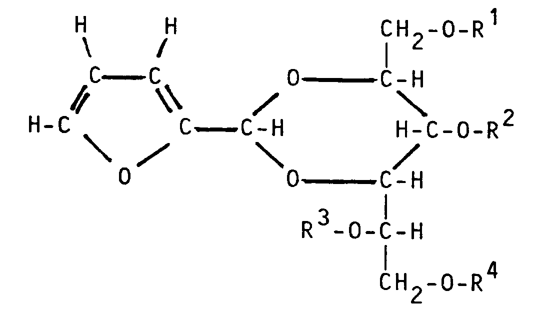 und ist in den Chemical Abstracts unter der Registraturnummer 7089-59-0 abzurufen.

R¹⁻⁴ bedeuten erfindungsgemäß unabhängig voneinander H, Methyl- und/oder Ethylgruppen. Erfindungsgemäß besonders bevorzugt sind R¹⁻⁴ identisch und stellen H oder Methylgruppen dar (2,4-Monofurfuryliden-tetra-O-methyl-sorbitol).

Die Konfiguration der vier asymmetrischen Kohlenstoffatome im vom Sorbitol abgeleiteten Molekülteil entspricht bevorzugt der der Kohlenstoffatome im natürlichen Sorbitol, obgleich auch die anderen Stereoisomere grundsätzlich vorteilhaft sind.

Die Konfiguration des asymmetrischen Kohlenstoffatoms der Furfurylidengruppe kann gleichermaßen vorteilhaft R oder S sein. Ein Racemat aus R- und S-Konfiguration, bezogen auf dieses Kohlenstoffatom, ist erfindungsgemäß von besonderem Vorteil.

Die erfindungsgemäßen Antioxidantien können vorteilhaft aus der Gruppe der üblichen kosmetischen und dermatologischen Antioxidantien gewählt werden, insbesondere aus der Gruppe bestehend aus Tocopherolen und deren Derivaten, besonders α-Tocopherol bzw. α-Tocopherylestern, insbesondere α-Tocopherylacetat, ferner Sesamol, Gallensäurederivaten wie Methyl-, Ethyl-, Propyl-, Amyl-, Butyl- und Laurylgallat, dem Konyferylbenzoat des Benzoeharzes, Nordihydroguajakharzsäure, Nordihydroguajaretsäure, Butylhydroxyanisol, Butylhydroxytoluol, Ascorbinsäure, Citronensäure, Phosphorsäure, Lecithin, Trihydroxybutyrophenon, Carotinen, Vitamin-A und dessen Derivaten, insbesondere Retinylpalmitat, Ascorbinsäure, Ascorbylpalmitat, Dilaurylthiodipropionat, Distearylthiodipropionat, Monoisopropylcitrat, Thiodipropionsäure und EDTA sowie EDTA-Derivaten.

Bevorzugt ist, die erfindungsgemäßen Antioxidantien aus der Gruppe der Tocopherole und deren Derivaten zu wählen.

Die Tocopherole, auch Vitamin E genannt, leiten sich vom Grundkörper Tocol ((2-Methyl-2-(4,8,12-trimethyltridecyl)-chroman-6-ol) ab und sind durch folgende Strukturen gekennzeichnet:

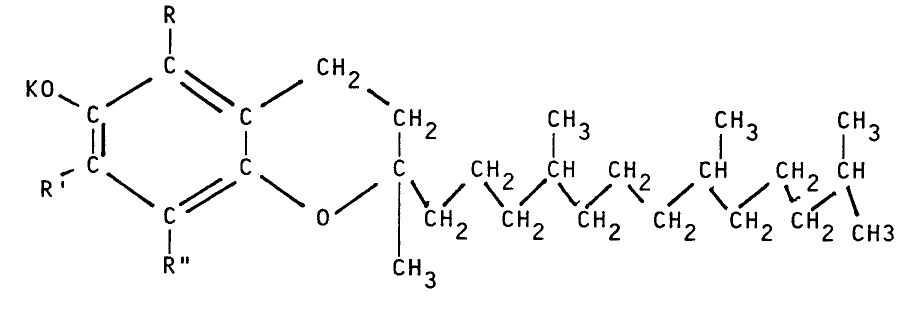

Dabei stellt K entweder H oder einen Acylrest dar, R, R' und R'' bedeuten unabhängig voneinander H oder eine Methylgruppe, z.B.:

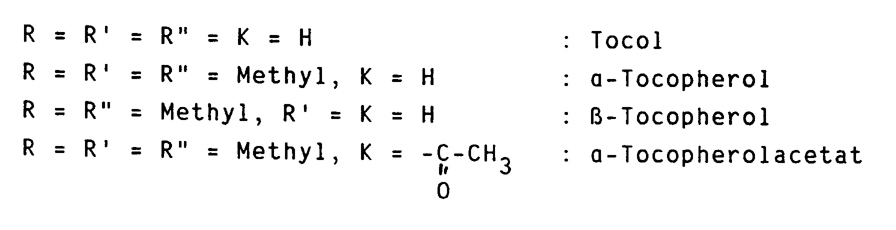

und weitere Varianten. In diesen Estern gilt für den Acylrest K: 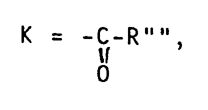 wobei R'''' einen Alkyl oder Alkenylrest mit 1 bis 21 Atomen darstellen kann. Besonders bevorzugt ist R'''' = Methyl.

Dem natürlich am häufigsten vorkommenden und bedeutendsten α-Tocopherol kommt die Konfiguration 2R,4'R,8'R zu. Es wird gelegentlich auch RRR-α-Tocopherol genannt.

Die erfindungsgemäß bevorzugten Tocopherolderivate sind das α-Tocopherol und seine Ester, insbesondere das α-Tocopherylacetat.

Es ist aus der EP-Patentanmeldungsschrift 345 362 bekannt, 2,4-Furfurylidensorbitol in kosmetischen und dermatologischen Formulierungen einzusetzen in solchen Formulierungen dient es in erster Linie als abfangendes Agens für freie Radikale, welche unter anderem durch UV-Strahlung hervorgerufen werden können.

Ferner ist aus den US-Patentschriften 4,144,325 und 4,248,861 sowie aus zahlreichen anderen Dokumenten bekannt, Vitamin E in kosmetischen und dermatologischen Lichtschutzformulierungen einzusetzen.

Es war indes nicht vorherzusehen gewesen, daß die erfindungsgemäßen Kombinationen aus 2,4-O-Furfurylidensorbitol nd dessen Alkylether sowie mindestens einer Substanz, gewählt aus der Gruppe der Antioxidantien, insbesondere der Tocopherole und Tocopherylester, in synergistischer Weise besser als die jeweiligen Einzelsubstanzen
- besser gegen Schädigung durch UV-Strahlung schützen
- besser als Antioxidans wirken
- besser als Radikalfänger wirken
- besser die Bindung von schädlichen Photoprodukten an Lipide, DNS und Proteine verhindern
würden. Ferner war nicht vorherzusehen gewesen, daß die erfindungsgemäßen Wirkstoffkombinationen oder die erfindungsgemäßen kosmetischen oder dermatolgischen Zubereitungen
- für die Anwendung genügend hohe Stabilität aufweisen
- zu hautverträglichen Produkten führen
- nicht in die hauteigene Mikroorganismenflora eingreifen
- der lichtinduzierten Hautalterung entgegenwirken würden.

Insbesondere war nicht vorherzusehen, daß die erfindungsgemäßen Wirkstoffkombinationen oder die erfindungsgemäßen kosmetischen oder dermatolgischen Zubereitungen sich durch eine ausgeprägte verzögerte Wirkung ("Retard-Wirkung") auszeichnen würden.

Darüber hinaus sind die erfindungsgemäßen Wirkstoffkombinationen oder die erfindungsgemäßen kosmetischen oder dermatolgischen Zubereitungen in besonderem Maße zur Penetration in tiefergelegene Hautschichten geeignet, wo sie in vorteilhafter Weise ihre Wirkung entfalten können.

Weiterhin eignen sich die erfindungsgemäßen Wirkstoffkombinationen oder die erfindungsgemäßen kosmetischen oder dermatolgischen Zubereitungen erstaunlicherweise zur gezielten Prophylaxe und/oder Behandlung UV-induzierter Hautschäden.

Erfindungsgemäß ist demnach auch die Verwendung der erfindungsgemäßen Wirkstoffkombinationen oder die erfindungsgemäßen kosmetischen oder dermatolgischen Zubereitungen zum Schutze der Haut gegen schädlichen Einfluß von ultraviolettem Licht.

In erstaunlicher Weise hat sich herausgestellt, daß die erfindungsgemäßen Wirkstoffkombinationen oder die erfindungsgemäßen kosmetischen oder dermatolgischen Zubereitungen in der Lage sind, photochemisch erzeugte Radikale abzufangen, vor photochemisch induzierten unkontrollierten Oxidationsprozessen zu schützen und sogar Singulettsauerstoff zu "quenchen", also durch einen physikochemischen Vorgang in den Triplettgrundzustand zu überführen. Stoffe mit dieser Eigenschaft werden auch als "Quenching Agents" bezeichnet.

Erfindungsgemäß ist daher auch die Verwendung der erfindungsgemäßen Wirkstoffkombinationen als Radikalfänger, Antioxidans und/oder Quenching Agent für photochemisch erzeugte reaktive Substanzen wie Singulettsauerstoff.

Die erfindungsgemäßen kosmetischen und/oder dermatologischen Formulierungen können wie üblich zusammengesetzt sein und zur Behandlung der Haut und/oder der Haare im Sinne einer dermatologischen Behandlung oder einer Behandlung im Sinne der pflegenden Kosmetik dienen. Sie können aber auch in Schminkprodukten in der dekorativen Kosmetik eingesetzt werden. Sie enthalten bevorzugt 0,01 Gew.-% bis 10 Gew.-%, insbesondere aber 0,1 Gew.-% bis 6 Gew.-%, bezogen auf das Gesamtgewicht einer oder mehrerer Substanzen aus der Gruppe bestehend aus 2,4-O-Furfurylidensorbitol und dessen Alkylether sowie mindestens einer Substanz, gewählt aus der Gruppe des Tocopherols und seiner Ester.

Zur Anwendung werden die erfindungsgemäßen Wirkstoffkombinationen oder die erfindungsgemäßen kosmetischen oder dermatolgischen Zubereitungen in der für Kosmetika und Dermatika üblichen Weise auf die Haut und/oder die Haare in ausreichender Menge aufgebracht.

Besonders bevorzugt sind solche kosmetische und dermatologische Zubereitungen, die in der Form eines Sonnenschutzmittels vorliegen. Vorteilhaft enthalten diese zusätzlich mindestens einen UVA-Filter und/oder mindestens einen UVB-Filter und/oder mindestens ein anorganisches Pigment.

Erfindungsgemäße kosmetische und/oder dermatologische Zubereitungen zum Schutze der Haut vor UV-Strahlen können in verschiedenen Formen vorliegen, wie sie z.B. üblicherweise für diesen Typ von Zubereitungen eingesetzt werden. So können sie z.B. eine Lösung, eine Emulsion vom Typ Wasser-in-Öl (W/O) oder vom Typ Öl-in-Wasser (O/W), oder eine multiple Emulsionen, beispielsweise vom Typ Wasser-in-Öl-in-Wasser (W/O/W), ein Gel, eine Hydrodispersion, einen festen Stift oder auch ein Aerosol darstellen.

Die erfindungsgemäßen kosmetischen Zubereitungen können kosmetische Hilfsstoffe enthalten, wie sie üblicherweise in solchen Zubereitungen verwendet werden, z.B. Konservierungsmittel, Bakterizide, Parfüme, Mittel zum Verhindern des Schäumens, Farbstoffe, Pigmente, die eine färbende Wirkung haben, Verdickungsmittel, oberflächenaktive Substanzen, Emulgatoren, weichmachende Substanzen, anfeuchtende und/oder feuchhaltende Substanzen, Fette, Öle, Wachse oder andere übliche Bestandteile einer kosmetischen Formulierung wie Alkohole, Polyole, Polymere, Schaumstabilisatoren, Elektrolyte, organische Lösungsmittel oder Silikonderivate.

Sofern die kosmetische oder dermatologische Zubereitung eine Lösung oder Lotion darstellt, können als Lösungsmittel verwendet werden:
- Wasser oder wäßrige Lösungen;
- Öle, wie Triglyceride der Caprin- oder der Caprylsäure, vorzugsweise aber Rizinusöl;
- Mineralöle;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte.

Insbesondere werden Gemische der vorstehend genannten Lösungsmittel verwendet. Bei alkoholischen Lösungsmitteln kann Wasser ein weiterer Bestandteil sein.

Emulsionen gemäß der Erfindung z.B. in Form einer Sonnenschutzcreme, einer Sonnenschutzlotion oder einer Sonnenschutzmilch sind vorteilhaft und enthalten z.B. die genannten Fette, Öle, Wachse und anderen Fettkörper, sowie Wasser und einen Emulgator, wie er üblicherweise für einen solchen Typ der Formulierung verwendet wird.

Auch Hydrodispersionen stellen vorteilhafte Verkörperungen der vorliegenden Erfindung dar.

Gele gemäß der Erfindung enthalten üblicherweise Alkohole niedriger C-Zahl, z.B. Ethanol, Isopropanol, 1,2-Propandiol, Glycerin und Wasser bzw. ein vorstehend genanntes Öl in Gegenwart eines Verdickungsmittels, das bei ölig-alkoholischen Gelen vorzugsweise Siliciumdioxid oder ein Aluminiumsilikat, bei wäßrig-alkoholischen oder alkoholischen Gelen vorzugweise ein Polyacrylat ist.

Feste Stifte gemäß der Erfindung enthalten z.B. natürliche oder synthetische Wachse, Fettalkohole oder Fettsäureester. Bevorzugt werden Lippenpflegestifte.

Als Treibmittel für erfindungsgemäße, aus Aerosolbehältern versprühbare kosmetische oder dermatologische Zubereitungen sind die üblichen bekannten leichtflüchtigen, verflüssigten Treibmittel, beispielsweise Kohlenwasserstoffe (Propan, Butan, Isobutan) geeignet, die allein oder in Mischung miteinander eingesetzt werden können. Auch Druckluft ist vorteilhaft zu verwenden.

Natürlich weiß der Fachmann, daß es an sich nichttoxische Treibgase gibt, die grundsätzlich für die vorliegende Erfindung geeignet wären, auf die aber dennoch wegen bedenklicher Wirkung auf die Umwelt oder sonstiger Begleitumstände verzichtet werden sollte, insbesondere Fluorkohlenwasserstoffe und Fluorchlorkohlenwasserstoffe (FCKW).

Vorteilhafte erfindungsgemäße kosmetische und dermatologische Zubereitungen zum Schutz der Haut enthalten die erfindungsgemäßen Wirkstoffkombinationen in Mengen von 0,01 - 10 Gew.-%, vorzugsweise in Mengen von 0,1 - 6 Gew.-%, insbesondere aber 0,1 - 4 Gew.-% bezogen auf das Gesamtgewicht der Zubereitung.

Bevorzugt können die erfindungsgemäßen Zubereitungen außerdem Substanzen enthalten, die UV-Strahlung im UVB-Bereich absorbieren, wobei die Gesamtmenge der Filtersubstanzen z.B. 0,1 Gew.-% bis 30 Gew.-%, vorzugsweise 0,5 bis 10 Gew.-%, insbesondere 1 bis 6 Gew.-% beträgt, bezogen auf das Gesamtgewicht der Zubereitung, um kosmetische Zubereitungen zur Verfügung zu stellen, die die Haut vor dem gesamten Bereich der ultravioletten Strahlung schützen. Sie können auch als Sonnenschutzmittel dienen.

Die UVB-Filter können öllöslich oder wasserlöslich sein. Erfindungsgemäß vorteilhaft können als öllösliche Substanzen verwendet werden:
- 3-Benzylidencampher-Derivate, vorzugsweise 3-(4-Methylbenzyliden)campher, 3-Benzylidencampher;
- 4-Aminobenzoesäure-Derivate, vorzugsweise 4-(Dimethylamino)-benzoesäure(2-ethylhexyl)ester, 4-(Dimethylamino)benzoesäureamylester;
- Ester der Zimtsäure, vorzugsweise 4-Methoxyzimtsäure(2-ethylhexyl)ester, 4-Methoxyzimtsäureisopentylester;
- Ester der Salicylsäure, vorzugsweise Salicylsäure(2-ethylhexyl)ester, Salicylsäure(4-isopropylbenzyl)ester, Salicylsäurehomomenthylester;
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
- Ester der Benzalmalonsäure, vorzugsweise 4-Methoxybenzalmalonsäuredi(2-ethylhexyl)ester;
- 2,4,6-Trianilino-(p-carbo-2'-ethyl-1'-hexyloxy)-1,3,5-triazin.

Erfindungsgemäß vorteilhaft können als wasserlösliche Substanzen verwendet werden:
- Salze der 2-Phenylbenzimidazol-5-sulfonsäure wie ihr Natrium-, Kalium- oder ihr Triethanolammonium-Salz, sowie die Sulfonsäure selbst;
- Sulfonsäure-Derivate von Benzophenonen, vorzugsweise 2⁻Hydroxy-4-methoxybenzophenon-5-sulfonsäure und ihre Salze;
- Sulfonsäure-Derivate des 3-Benzylidencamphers, wie z.B. 4-(2-Oxo-3-bornylidenmethyl)benzolsulfonsäure, 2-Methyl-5-(2-oxo-3-bornylidenmethyl)sulfosäure und ihre Salze.

Die Liste der genannten UVB-Filter, die in Kombination mit den erfindungsgemäßen Wirkstoffkombinationen verwendet werden können, soll selbstverständlich nicht limitierend sein.

Gegenstand der Erfindung ist auch die Kombination erfindungsgemäßer Wirkstoffkombinationen mit einem oder mehreren UVB-Filtern bzw. erfindungsgemäße kosmetische oder dermatologische Zubereitungen, welches auch einen oder mehrere UVB-Filter enthalten.

Es kann auch von Vorteil sein, erfindungsgemäße Wirkstoffkombinationen mit UVA-Filtern zu kombinieren, die bisher üblicherweise in kosmetischen und/oder dermatologischen enthalten sind. Bei diesen Substanzen handelt es sich vorzugsweise um Derivate des Dibenzoylmethans, insbesondere um 1-(4'-tert.Butylphenyl)-3-(4'-methoxyphenyl)propan-1,3-dion und um 1-Phenyl-3-(4'-isopropylphenyl)propan-1,3-dion. Auch diese Kombinationen bzw. Zubereitungen, die diese Kombinationen enthalten, sind Gegenstand der Erfindung. Es können die für die UVB-Kombination verwendeten Mengen eingesetzt werden.

Ferner werden vorteilhafte Zubereitungen erhalten, wenn die erfindungsgemäßen Wirkstoffkombinationen mit UVA- und UVB-Filtern kombiniert werden.

Erfindungsgemäße kosmetische und/oder dermatologische Lichtschutzzubereitungen, können auch anorganische Pigmente enthalten, die üblicherweise in der Kosmetik zum Schutze der Haut vor UV-Strahlen verwendet werden. Dabei handelt es sich um Oxide des Titans, Zinks, Eisens, Zirkoniums, Siliciums, Mangans, Aluminiums, Cers und Mischungen davon, sowie Abwandlungen, bei denen die Oxide die aktiven Agentien sind. Besonders bevorzugt handelt es sich um Pigmente auf der Basis von Titandioxid.

Auch diese Kombinationen von UVA-Filter und/oder UVB-Filter und Pigment bzw. Zubereitungen, die diese Kombination enthalten, sind Gegenstand der Erfindung. Es können die für die vorstehenden Kombinationen genannten Mengen verwendet werden.

Bei kosmetischen Zubereitungen zum Schutz der Haare vor UV-Strahlen gemäß der Erfindung handelt es sich beispielsweise um Shampoonierungsmittel, Zubereitungen, die beim Spülen der Haare vor oder nach der Shampoonierung, vor oder nach der Dauerwellbehandlung, vor oder nach der Färbung oder Entfärbung der Haare angewendet werden, um Zubereitungen zum Fönen oder Einlegen der Haare, Zubereitungen zum Färben oder Entfärben, um eine Frisier- und Behandlungslotion, einen Haarlack oder um Dauerwellmittel. Die kosmetischen Zubereitungen enthalten Wirkstoffe und Hilfsstoffe, wie sie üblicherweise für diesen Typ von Zubereitungen zur Haarpflege und Haarbehandlung verwendet werden. Als Hilfsstoffe dienen Konservierungsmittel, oberflächenaktive Substanzen, Substanzen zum Verhindern des Schäumens, Emulgatoren, Verdickungsmittel, Fette, Öle, Wachse, organische Lösungsmittel, Bakterizide, Parfüme, Farbstoffe oder Pigmente, deren Aufgabe es ist, die Haare oder die Zubereitung selbst zu färben, Elektrolyte, Zubereitungen gegen das Fetten der Haare.

Kosmetische Zubereitungen, die ein Shampoonierungsmittel darstellen, enthalten vorzugsweise mindestens eine anionische, nicht-ionische oder amphotere oberflächenaktive Substanz oder Gemische daraus, eine erfindungsgemäße Wirkstoffkombination im wäßrigen Medium und Hilfsmittel, wie sie üblicherweise dafür verwendet werden. Die oberflächenaktive Substanz kann in einer Konzentration zwischen 1 Gew.-% und 50 Gew.-% in dem Shampoonierungsmittel vorliegen.

Liegt die erfindungsgemäße kosmetische oder dermatologische Zubereitung in Form einer Lotion vor, die ausgespült und z.B. vor oder nach der Entfärbung, vor oder nach der Shampoonierung, zwischen zwei Shampoonierungsschritten, vor oder nach der Dauerwellbehandlung angewendet wird, so handelt es sich dabei z.B. um wäßrige oder wäßrig-alkoholische Lösungen, die gegebenenfalls oberflächenaktive Substanzen enthalten, bevorzugt nicht-ionische oder kationische oberflächenaktive Substanzen, deren Konzentration zwischen 0,1 und 10 Gew.-%, vorzugsweise zwischen 0,2 und 5 Gew.-%, liegen kann. Diese kosmetische oder dermatologische Zubereitung kann auch ein Aerosol mit den üblicherweise dafür verwendeten Hilfsmitteln darstellen.

Eine kosmetische Zubereitung in Form einer Lotion, die nicht ausgespült wird, insbesondere eine Lotion zum Einlegen der Haare, eine Lotion, die beim Fönen der Haare verwendet wird, eine Frisier- und Behandlungslotion, stellt im allgemeinen eine wäßrige, alkoholische oder wäßrig-alkoholische Lösung dar und enthält mindestens ein kationisches, anionisches, nicht-ionisches oder amphoteres Polymer oder auch Gemische derselben, sowie eine erfindungsgemäße Wirkstoffkombination. Die Menge der verwendeten Polymeren liegt z.B. zwischen 0,1 und 10 Gew.-%, bevorzugt zwischen 0,1 und 3 Gew.-%.

Erfindungsgemäße kosmetische und dermatologische Zubereitungen zur Behandlung und Pflege der Haare, die eine erfindungsgemaße Wirkstoffkombination enthalten, können als Emulsionen vorliegen, die vom nicht-ionischen oder anionischen Typ sind. Nicht-ionische Emulsionen enthalten neben Wasser Öle oder Fettalkohole, die beispielsweise auch polyethoxyliert oder polypropoxyliert sein können, oder auch Gemische aus den beiden organischen Komponenten. Diese Emulsionen enthalten gegebenenfalls kationische oberflächenaktive Substanzen. Anionische Emulsionen sind vorzugsweise vom Typ einer Seife.

Kosmetische und dermatologische Zubereitungen zur Behandlung und Pflege der Haare können als Gele vorliegen, die neben einer erfindungsgemäßen Wirkstoffkombination und dafür üblicherweise verwendeten Lösungsmitteln noch organische Verdickungsmittel, z.B. Gummiarabikum, Xanthangummi, Natriumalginat, Cellulose-Derivate, vorzugsweise Methylcellulose, Hydroxymethylcellulose, Hydroxyethylcellulose, Hydroxypropylcellulose, Hydroxypropylmethylcellulose oder anorganische Verdickungsmittel, z. B. Aluminiumsilikate wie beispielsweise Bentonite, oder ein Gemisch aus Polyethylenglykol und Polyethylenglykolstearat oder -distearat, enthalten. Das Verdickungsmittel ist in dem Gel z.B. in einer Menge zwischen 0,1 und 30 Gew.-%, bevorzugt zwischen 0,5 und 15 Gew.-%, enthalten.

Vorzugsweise beträgt die Menge der erfindungsgemäßen Wirkstoffkombinationen in einem für die Haare bestimmten Mittel 0,01 Gew.-% bis 10 Gew.%, insbesondere 0,5 Gew.-% bis 5 Gew.-%, bezogen auf das Gesamtgewicht der Zubereitungen.

Die vorliegende Erfindung umfaßt auch ein Verfahren zum Schutze der Haut und der Haare vor UVA- und UVB-Strahlung, das dadurch gekennzeichnet ist, daß man eine kosmetische oder dermatologische Zubereitung, eine erfindungsgemäße Wirkstoffkombination enthaltend, in ausreichender Menge auf die Haut oder Haare aufbringt, sowie die Verwendung dieser Wirkstoffkombinationen für diese Zwecke.

Ebenso umfaßt die vorliegende Erfindung auch ein Verfahren zum Schutz farbloser oder gefärbter kosmetischer und dermatologischer Zubereitungen gegen UVA- und UVB-Strahlen sowie diese Zubereitungen selbst, bei denen es sich z.B. um vorstehend genannte Zubereitungen zur Behandlung und Pflege der Haare, insbesondere um Haarfärbemittel, Haarlacke, Shampoonierungsmittel, Farbshampoonierungsmittel, um Schminkprodukte wie z.B. Nagellacke, Lippenstifte, Teintgrundlagen, Cremes zur Behandlung der Haut oder um sämtliche anderen kosmetischen Zubereitungen handelt, deren Bestandteile Stabilitätsprobleme aufgrund von Licht bei der Lagerung mit sich bringen können, dadurch gekennzeichnet, daß den kosmetischen oder dermatologischen Zube reitungen eine erfindungsgemäße Wirkstoffkombination in für die Stabilisierung gegenüber Licht ausreichender Menge zugesetzt wird.

Vorzugsweise beträgt die Menge der erfindungsgemäßen Wirkstoffkombinationen in diesen Zubereitungen 0,01 Gew.-% bis 10 Gew.-%, insbesondere 0,1 Gew.-% bis 3 Gew.-%, bezogen auf das Gesamtgewicht der Zubereitungen.

Gegenstand der Erfindung ist auch das Verfahren zur Herstellung der erfindungsgemäßen kosmetischen Zubereitungen, das dadurch gekennzeichnet ist, daß man in an sich bekannter Weise erfindungsgemäße Wirkstoffkombinationen in kosmetische oder dermatologische Formulierungen einarbeitet.

Die nachfolgenden Beispiele sollen die vorliegende Erfindung verdeutlichen, ohne sie einzuschränken. Die Angaben betreffen Gewichtsprozente, bezogen auf das Gesamtgewicht der Zubereitungen.

Die nachfolgenden Angaben zu den Zusammensetzungen der Wirkstoffkombinationen I - V beziehen sich auf das Gesamtgewicht der Wirkstoffkombinationen.

| Wirkstoffkombination I | |
|---|---|
| | Gew.-% |
| Furfurylidensorbitol | 10,00 |
| Vitamin E-Acetat | 90,00 |

| Wirkstoffkombination II | |
|---|---|
| | Gew.-% |
| Furfurylidensorbitol | 25,00 |
| Vitamin E-Acetat | 75,00 |

| Wirkstoffkombination III | |
|---|---|
| | Gew.-% |
| Furfurylidensorbitol | 50,00 |
| Vitamin E-Acetat | 50,00 |

| Wirkstoffkombination IV | |
|---|---|
| | Gew.-% |
| Furfurylidensorbitol | 75,00 |
| Vitamin E-Acetat | 25,00 |

| Wirkstoffkombination V | |
|---|---|
| | Gew.-% |
| Furfurylidensorbitol | 90,00 |
| Vitamin E-Acetat | 10,00 |

| Beispiel 1 | |
|---|---|
| | Gew.-% |
| Cyclomethicone | 2,000 |
| Cetyldimethicone Copolyol | 0,200 |
| PEG-22-Dodecyl Copolymer | 3,000 |
| Paraffinöl (DAB 9) | 2,000 |
| Caprylsäure-/Caprinsäure Triglycerid | 5,800 |
| Octylmethoxycinnamat | 5,800 |
| Butyl-methoxy-dibenzoylmethan | 4,000 |
| Wirkstoffkombination I | 0,300 |
| ZnSO₄ | 0,700 |
| Na₄EDTA | 0,300 |
| Parfum, Konservierungsmittel, Farbstoffe | nach Belieben |
| H₂O VES | ad 100,000 |

| Beispiel 2 | |
|---|---|
| | Gew.-% |
| Cyclomethicone | 2,000 |
| Cetyldimethicone Copolyol | 0,200 |
| PEG-22-Dodecyl Copolymer | 3,000 |
| Paraffinöl (DAB 9) | 2,000 |
| Caprylsäure-/Caprinsäure Triglycerid | 5,800 |
| Octylmethoxycinnamat | 5,800 |
| Butyl-methoxy-dibenzoylmethan | 4,000 |
| Wirkstoffkombination II | 0,500 |
| ZnSO₄ | 0,700 |
| Na₄EDTA | 0,300 |
| Parfum, Konservierungsmittel, Farbstoffe | nach Belieben |
| H₂O VES | ad 100,000 |

| Beispiel 3 | |
|---|---|
| | Gew.-% |
| Cyclomethicone | 2,000 |
| Cetyldimethicone Copolyol | 0,200 |
| PEG-22-Dodecyl Copolymer | 3,000 |
| Paraffinöl (DAB 9) | 2,000 |
| Caprylsäure-/Caprinsäure Triglycerid | 5,800 |
| Octylmethoxycinnamat | 5,800 |
| Butyl-methoxy-dibenzoylmethan | 4,000 |
| Wirkstoffkombination III | 2,500 |
| ZnSO₄ | 0,700 |
| Na₄EDTA | 0,300 |
| Parfum, Konservierungsmittel, Farbstoffe | nach Belieben |
| H₂O VES | ad 100,000 |

| Beispiel 4 | |
|---|---|
| | Gew.-% |
| Cyclomethicone | 2,000 |
| Cetyldimethicone Copolyol | 0,200 |
| PEG-22-Dodecyl Copolymer | 3,000 |
| Paraffinöl (DAB 9) | 2,000 |
| Caprylsäure-/Caprinsäure Triglycerid | 5,800 |
| Octylmethoxycinnamat | 5,800 |
| Butyl-methoxy-dibenzoylmethan | 4,000 |
| Wirkstoffkombination IV | 3,000 |
| ZnSO₄ | 0,700 |
| Na₄EDTA | 0,300 |
| Parfum, Konservierungsmittel, Farbstoffe | nach Belieben |
| H₂O VES | ad 100,000 |

| Beispiel 5 | |
|---|---|
| Cyclomethicone | 2,000 |
| Cetearylalkohol + PEG-40-hydriertes Rizinusöl + Natrium Cetearylsulfat | 2,500 |
| Glyceryllanolat | 1,000 |
| Caprylsäure-/Caprinsäure Triglycerid | 0,100 |
| Laurylmethicon Copolyol | 2,000 |
| Octylstearat | 3,000 |
| Rizinusöl | 4,000 |
| Glycerin | 3,000 |
| Acrylamid/Natriumacrylat Copolymer | 0,300 |
| Hydroxypropylmethylcellulose | 0,300 |
| Octylmethoxycinnamat | 5,000 |
| Butyl-methoxy-dibenzoylmethan | 0,500 |
| Wirkstoffkombination V | 1,750 |
| Na_{3HEDTA} | 1,500 |
| Parfum, Konservierungsmittel, Farbstoffe | nach Belieben |
| H₂O VES | ad 100,000 |

| Beispiel 6 | |
|---|---|
| Cyclomethicone | 2,000 |
| Cetearylalkohol + PEG-40-hydriertes Rizinusöl + Natrium Cetearylsulfat | 2,500 |
| Glyceryllanolat | 1,000 |
| Caprylsäure-/Caprinsäure Triglycerid | 0,100 |
| Laurylmethicon Copolyol | 2,000 |
| Octylstearat | 3,000 |
| Rizinusöl | 4,000 |
| Glycerin | 3,000 |
| Acrylamid/Natriumacrylat Copolymer | 0,300 |
| Hydroxypropylmethylcellulose | 0,300 |
| Octylmethoxycinnamat | 5,000 |
| Butyl-methoxy-dibenzoylmethan | 0,750 |
| Wirkstoffkombination I | 2,500 |
| Na_{3HEDTA} | 1,500 |
| Parfum, Konservierungsmittel, Farbstoffe | nach Belieben |
| H₂O VES | ad 100,000 |

| Beispiel 7 | |
|---|---|
| Cyclomethicone | 2,000 |
| Cetearylalkohol + PEG-40-hydriertes Rizinusöl + Natrium Cetearylsulfat | 2,500 |
| Glyceryllanolat | 1,000 |
| Caprylsäure-/Caprinsäure Triglycerid | 0,100 |
| Laurylmethicon Copolyol | 2,000 |
| Octylstearat | 3,000 |
| Rizinusöl | 4,000 |
| Glycerin | 3,000 |
| Acrylamid/Natriumacrylat Copolymer | 0,300 |
| Hydroxypropylmethylcellulose | 0,300 |
| Octylmethoxycinnamat | 5,000 |
| Butyl-methoxy-dibenzoylmethan | 1,000 |
| Wirkstoffkombination II | 0,700 |
| Na_{3HEDTA} | 1,500 |
| Parfum, Konservierungsmittel, Farbstoffe | nach Belieben |
| H₂O VES | ad 100,000 |

| Beispiel 8 | |
|---|---|
| Cyclomethicone | 2,000 |
| Cetearylalkohol + PEG-40-hydriertes Rizinusöl + Natrium Cetearylsulfat | 2,500 |
| Glyceryllanolat | 1,000 |
| Caprylsäure-/Caprinsäure Triglycerid | 0,100 |
| Laurylmethicon Copolyol | 2,000 |
| Octylstearat | 3,000 |
| Rizinusöl | 4,000 |
| Glycerin | 3,000 |
| Acrylamid/Natriumacrylat Copolymer | 0,300 |
| Hydroxypropylmethylcellulose | 0,300 |
| Octylmethoxycinnamat | 5,000 |
| Butyl-methoxy-dibenzoylmethan | 0,500 |
| Wirkstoffkombination III | 1,600 |
| Na_{3HEDTA} | 1,500 |
| Parfum, Konservierungsmittel, Farbstoffe | nach Belieben |
| H₂O VES | ad 100,000 |

## Patentansprüche

1. Kosmetische oder dermatologische Zubereitungen zum Schutze der Haut gegen Oxidationsprozesse, gekennzeichnet durch einen Gehalt an Wirkstoffkombinationen, bestehend aus mindestens einer Substanz, gewählt aus der Gruppe aus 2,4-O-Furfurylidensorbitol und dessen Alkylethern sowie mindestens 0,09 Gew.-%, bezogen auf das Gesamtgewicht der Zubereitungen, einer oder mehrerer Substanzen, gewählt aus der Gruppe der Tocopherole und Tocopherylester, insbesondere aus der Gruppe α-Tocopherol und α-Tocopherylacetat..

2. Zubereitungen nach Anspruch 1, dadurch gekennzeichnet, daß der Alkylether des 2,4-O-Furfurylidensorbitols das 2,4-Monofurfuryliden-tetra-O-methyl-sorbitol ist.

3. Zubereitungen nach Anspruch 1, dadurch gekennzeichnet, daß als zusätzliche kosmetisch oder pharmazeutisch akzeptablen Antioxidantien gewählt werden aus der Gruppe bestehend aus Sesamol, Gallensäurederivaten wie Methyl-, Ethyl-, Propyl-, Amyl-, Butyl- und Laurylgallat, dem Konyferylbenzoat des Benzoeharzes, Nordihydroguajakharzsäure, Nordihydroguajaretsäure, Butylhydroxyanisol, Butylhydroxytoluol, Ascorbinsäure, Citronensäure, Phosphorsäure, Lecithin, Trihydroxybutyrophenon, Carotinen, Vitamin-A und dessen Derivaten, insbesondere Retinylpalmitat, Ascorbinsäure, Ascorbylpalmitat, Dilaurylthiodipropionat, Distearylthiodipropionat, Monoisopropylcitrat, Thiodipropionsäure und EDTA sowie EDTA-Derivaten.

4. Zubereitungen nach Anspruch 1, dadurch gekennzeichnet, daß sie Lichtschutzformulierungen darstellen.

5. Zubereitungen nach Anspruch 1, dadurch gekennzeichnet, daß der Gehalt an einer Wirkstoffkombination gewählt wird aus einem Konzentrationsbereich von 0,1 - 10 Gew.-%, vorzugsweise von 0,1 - 6 Gew.-%, insbesondere von 0,1 - 4 Gew.-%, jeweils bezogen auf das Gesamtgewicht der Lichtschutzformulierungen.

6. Zubereitungen nach Anspruch 1, dadurch gekennzeichnet, daß sie eine Lösung, eine Emulsion vom Typ Wasser-in-Öl (W/O) oder vom Typ Öl-in-Wasser (O/W), oder eine multiple Emulsionen, ein Gel, eine Hydrodispersion, einen festen Stift oder ein Aerosol darstellen.

## Claims

1. Cosmetic or dermatological formulations for the protection of the skin against oxidation processes, characterized by a content of active compound combinations comprising at least one substance chosen from the group consisting of 2,4-O-furfurylidenesorbitol and alkyl ethers thereof and at least 0.09% by weight, based on the total weight of the formulations, of one or more substances chosen from the group consisting of tocopherols and tocopheryl esters, in particular from the group consisting of α-tocopherol and α-tocopheryl acetate.

2. Formulations according to Claim 1, characterized in that the alkyl ether of 2,4-O-furfurylidenesorbitol is 2,4-monofurfurylidene-tetra-O-methyl-sorbitol.

3. Formulations according to Claim 1, characterized in that additional cosmetically or pharmaceutically acceptable antioxidants are chosen from the group consisting of sesamol, bile acid derivatives, such as methyl, ethyl, propyl, amyl, butyl and lauryl gallate, coniferyl benzoate of benzoin resin, nordihydroguaiacum resin acid, nordihydroguaiaretic acid, butyl hydroxyanisole, butyl hydroxytoluene, ascorbic acid, citric acid, phosphoric acid, lecithin, trihydroxybutyrophenone, carotenes, vitamin A and derivatives thereof, in particular retinyl palmitate, ascorbic acid, ascorbyl palmitate, dilauryl thiodipropionate, distearyl thiodipropionate, monoisopropyl citrate, thiodipropionic acid and EDTA and EDTA derivatives.

4. Formulations according to Claim 1, characterized in that they constitute light protection formulations.

5. Formulations according to Claim 1, characterized in that the content of an active compound combination is selected from a concentration range of 0.1 - 10% by weight, preferably of 0.1 - 6% by weight, in particular 0.1 - 4% by weight, in each case based on the total weight of the light protection formulations.

6. Formulations according to Claim 1, characterized in that they are a solution, an emulsion of the water-in-oil (W/O) type or of the oil-in-water (O/W) type, or a multiple emulsions [sic], a gel, a hydrodispersion, a solid stick or an aerosol.

## Revendications

1. Compositions cosmétiques ou dermatologiques pour la protection de la peau contre des processus d'oxydation, caractérisées par une teneur en associations de substances actives, constituées d'au moins une substance choisie parmi le 2,4-O-furfurylidènesorbitol et ses éthers alkliques, ainsi que d'au moins 0,09 % en poids, par rapport au poids des compositions, d'une ou plusieurs substances choisies parmi les tocophérols et esters de tocophéryle, en particulier parmi l'α-tocophérol et l'acétate d'α-tocophéryle.

2. Compositions selon la revendication 1, caractérisées en ce que l'éther alkylique du 2,4-O-furfurylidène-sorbitol est le 2,4-monofurfurylidène-tétra-O-méthylsorbitol.

3. Compositions selon la revendication 1, caractérisées en ce que l'on choisit comme antioxydants supplémentaires, cosmétiquement ou pharmaceutiquement acceptables, des antioxydants dans le groupe constitué par le sésamol, des dérivés de l'acide gallique tels que le gallate de méthyle, éthyle, propyle, amyle, butyle ou lauryle, le benzoate de coniféryle de la résine de benjoin, l'acide nordihydrogiuaiarésinique, l'acide nordihydroguaiarétique, le butylhydroxyanisole, le butylhydroxytoluène, l'acide ascorbique, l'acide citrique, l'acide phosphorique, la lécithine, la trihydroxybutyrophénone, le carotène, la vitamine A et ses dérivés, en particulier le palmitate de rétinyle, l'acide ascorbique, le palmitate d'ascorbyle, le thiodipropionate de dilauryle, le thiodipropionate de distéaryle, le citrate de mono-isopropyle, l'acide thiodipropionigue et l'EDTA ainsi que des dérivés d'EDTA.

4. Compositions selon la revendication 1, caractérisées en ce qu'elles représentent des formulations photoprotectrices.

5. Compositions selon la revendication 1, caractérisées en ce que la teneur en une association de substances actives est choisie dans une plage de concentrations allant de 0,1 à 10 % en poids, de préférence de 0,1 à 6 % en poids, en particulier de 0,1 à 4 % en poids, dans chaque cas par rapport au poids total des formulations photoprotectrices.

6. Compositions selon la revendication 1, caractérisées en ce qu'elles représentent une solution, une émulsion du type eau-dans-huile (E/H) ou du type huile-dans-eau (H/E) ou une émulsion multiple, un gel, une hydrodispersion, un bâton solide ou une composition pour aérosol.
